# EUROPEAN PATENT SPECIFICATION

(11) **EP 4 491 155 B1**
(45) Date of publication and mention of the grant of the patent: **26.08.2026**
(21) Application number: 24186534.4
(22) Date of filing: 04.07.2024
(51) Int. Cl.: A61F 2/24, A61F 2/962

(54) **DELIVERY SYSTEM INCLUDING A SPLIT CAPSULE**
ABGABESYSTEM MIT EINER GETEILTEN KAPSEL
SYSTÈME DE DISTRIBUTION COMPRENANT UNE CAPSULE FENDUE

(30) Priority: 12.07.2023 US 202363513144 P; 27.03.2024 US 202418617773
(43) Date of publication of application: 15.01.2025
(73) Proprietor: Medtronic Inc., Minneapolis, MN 55432 (US)
(72) Inventor: FEERICK, Emer, Ballybrit Galway (IE)
(74) Representative: Zimmermann & Partner Patentanwälte mbB

(56) References cited:
- EP-B1- 2 892 467
- EP-B1- 3 541 329
- WO-A1-2022/118316
- US-A1- 2010 249 907

## Description

### Cross-Reference to Related Applications

This application claims the benefit of U.S. Provisional Patent Application Serial No. 63/513,144, filed July 12, 2023.

### Field of the Invention

The present disclosure relates generally to delivery systems and devices for the delivery of prosthetic valves, and more particularly to a delivery systems including a split capsule and related components.

### Background

Natural heart valves, such as aortic valves, mitral valves, pulmonary valves and tricuspid valves, often become damaged by disease in such a manner that they fail to maintain blood flow in a single direction. A malfunctioning heart valve may be stenotic (i.e., heart valve leaflets fail to fully open) or regurgitant (i.e., heart valve leaflets fail to fully close and/or seal). Maintenance of blood flow in a single direction through the heart valve is important for proper flow, pressure and perfusion of blood through the body. Hence, a heart valve that does not function properly may noticeably impair the function of the heart.

Cardiac valve prostheses are well known in the treatment of heart disease to replace malfunctioning heart valves. Heart valve replacement generally has been accomplished by major open-heart surgery. This is a serious operation that requires general anesthesia, full cardiopulmonary bypass with complete cessation of cardiopulmonary activity, an extended hospitalization stay, and several more weeks to months of recuperation time. For some patients, open heart surgery is not an option because of the critical condition of the patient, advanced age, existing comorbidities, or other physical limitations.

EP 3 541 329 B1 describes cardiac valve delivery devices. WO 2022/118316 A1 describes a transluminal delivery system.

Recently, there has been increasing interest in minimally invasive and percutaneous replacement of cardiac valves, typically by way of catheterization. In minimally invasive procedures, a catheter is used to insert a mechanical or bioprosthetic valve in a lumen of a blood vessel via percutaneous entry through a distal blood vessel. However, the procedures of loading these catheters with mechanical or bioprosthetic valves have proven to be cumbersome, with issues such as catheter breakage, undesired disassembly, or device failure resulting from the procedures of loading said mechanical or bioprosthetic valve into the catheter. Further, when delivering such a heart valve prosthesis, it may be beneficial to be able to recapture the heart valve prosthesis after partial deployment thereof to improve accuracy in deployment location.

Thus, there is a continuing need for improved transcatheter delivery systems and methods of implanting medical devices.

### SUMMARY

According to the invention a delivery system for delivering a device to a body lumen comprises an inner shaft coupled to a distal tip, and an outer sheath slidingly disposed over the inner shaft and configured to hold the device in a radially compressed configuration. The outer sheath has a proximal capsule with a distal end, and a distal capsule with a proximal end, such that the distal end of the proximal capsule is configured to overlap with the proximal end of the distal capsule to form an overlap region. A removable sleeve is configured to couple the proximal capsule to the distal capsule at the overlap region.

In an example hereof, in the delivery system of any of the previous or subsequent examples, the distal tip further includes a flange with a groove formed therein extending from a proximal end of the distal tip, and the distal capsule further includes a distal end configured to interlock with the groove of the flange of the distal tip.

In another example hereof, in the delivery system of any of the previous or subsequent examples, the distal end of the distal capsule further comprises an interlocking flange configured to be received by the groove of the flange of the distal tip.

In another example hereof, in the delivery system of any of the previous or subsequent examples, the interlocking flange comprises metal or rigid polymer.

In another example hereof, in the delivery system of any of the previous or subsequent examples, the distal end of the distal capsule further includes an interlock collar overlapping the distal end for securing the interlocking flange to the distal end of the distal capsule.

In another example hereof, in the delivery system of any of the previous or subsequent examples, the interlocking flange comprises a sloped face for sliding interaction with the flange of the distal tip, such that the flange of the distal tip is displaced from a longitudinal axis upon contacting the sloped face during the sliding interaction.

In another example hereof, in the delivery system of any of the previous or subsequent examples, the removable sleeve includes a plurality of tab protrusions for facilitating end user interaction.

In another example hereof, in the delivery system of any of the previous or subsequent examples, the removable sleeve includes at least one tab protrusion for facilitating end user interaction, and at least one perforation disposed along a length of the removable sleeve for removal of the removable sleeve from the overlap region.

In another example hereof, in the delivery system of any of the previous or subsequent examples, the at least one tab protrusion comprises a square, rectangular, circular, or oblong shaped protrusion extending from a body of the removable sleeve.

In another example hereof, in the delivery system of any of the previous or subsequent examples, the at least one tab protrusion comprises bumps, extensions, or other inconsistencies formed on a body of the removable sleeve.

In another example hereof, in the delivery system of any of the previous or subsequent examples, the distal end of the proximal capsule comprises a proximal overlap collar extending distally, and the proximal end of the distal capsule comprises a distal overlap collar extending proximally, and the proximal overlap collar is configured to overlap with the distal overlap collar to form the overlap region.

In another example hereof, in the delivery system of any of the previous or subsequent examples, the proximal overlap collar comprises a first thickness, and the distal overlap collar comprises a second thickness, such that a profile of the overlap region created by the overlapping of the proximal overlap collar and the distal overlap collar is no greater than a profile of a body portion of the proximal capsule and/or a body portion of the distal capsule.

In another example hereof, in the delivery system of any of the previous or subsequent examples, the outer sheath is translatable between an open configuration where the proximal capsule and the distal capsule are separated from each other, and a closed configuration where the distal end of the proximal capsule and the proximal end of the distal capsule are connected to each other forming the overlap region.

In another example hereof, a delivery system for delivering a device to a body lumen comprises an inner shaft coupled to a distal tip, and an outer sheath slidingly disposed over the inner shaft. The outer sheath is configured to hold the device in a radially compressed configuration. The outer sheath includes a proximal capsule and a distal capsule with a distal end configured to interlock with the distal tip.

In another example hereof, in the delivery system of any of the previous or subsequent examples, the distal end of the distal capsule further comprises an interlocking flange configured to be received by a groove of a flange extending from a proximal end of the distal tip.

In another example hereof, in the delivery system of any of the previous or subsequent examples, the interlocking flange comprises a metal or a rigid polymer.

In another example hereof, in the delivery system of any of the previous or subsequent examples, the distal end of the distal capsule further includes an interlock collar overlapping the distal end for securing the interlocking flange to the distal end of the distal capsule.

In another example hereof, in the delivery system of any of the previous or subsequent examples, the interlock collar radially compresses the interlocking flange to an outer surface of the distal end of the distal capsule.

In another example hereof, in the delivery system of any of the previous or subsequent examples, the interlocking flange comprises a sloped face for sliding interaction with the flange of the distal tip, such that the flange of the distal tip is displaced from a longitudinal axis upon contacting the sloped face during the sliding interaction.

In another example hereof, in the delivery system of any of the previous or subsequent examples, the distal tip is translatable between a loading configuration and a closed configuration, such that the distal tip is separated from the distal end of the distal capsule in the loading configuration, and the distal tip is secured to the distal end of the distal capsule in the closed configuration.

In another example hereof, in the delivery system of any of the previous or subsequent examples, the proximal capsule comprises a distal end, and the distal capsule comprises a proximal end, wherein the distal end of the proximal capsule is configured to overlap with the proximal end of the distal capsule to form an overlap region.

In another example hereof, the delivery system of any of the previous or subsequent examples further includes a removable sleeve configured to couple the proximal capsule to the distal capsule at the overlap region.

In another example hereof, in the delivery system of any of the previous or subsequent examples, the removable sleeve includes a plurality of tab protrusions for facilitating end user interaction.

In another example hereof, in the delivery system of any of the previous or subsequent examples, the removable sleeve includes at least one tab protrusion for facilitating end user interaction, and at least one perforation disposed along a length of the removable sleeve for removal of the removable sleeve from the overlap region.

In another example hereof, in the delivery system of any of the previous or subsequent examples, the at least one tab protrusion comprises a square, rectangular, circular, or oblong shaped protrusion extending from a body of the removable sleeve.

In another example hereof, in the delivery system of any of the previous or subsequent examples, the at least one tab protrusion comprises bumps, extensions, or other inconsistencies formed on a body of the removable sleeve.

In another example hereof, in the delivery system of any of the previous or subsequent examples, the outer sheath is translatable between an open configuration where the proximal capsule and the distal capsule are separated from each other, and a closed configuration where the distal end of the proximal capsule and the proximal end of the distal capsule are connected to each other forming the overlap region.

In another example hereof, a method of loading a device into a delivery system comprises attaching the device to the delivery system and radially compressing the device into an outer sheath of the delivery system. The outer sheath includes a proximal capsule having a distal end and a distal capsule having a proximal end. During the radially compressing step the distal end of the proximal capsule and the proximal end of the distal capsule overlap to form an overlap region and a removable sleeve is disposed around the overlap region coupling the proximal capsule to the distal capsule. The method furth includes removing the removable sleeve from the overlap region.

In another example hereof, the method of any of the previous or subsequent examples further includes interlocking a distal tip having a flange with an interlocking flange on a distal end of the distal capsule.

In another example hereof, the method of any of the previous or subsequent examples further includes capturing the interlocking flange of the distal end of the distal capsule via the groove of the flange of the distal tip.

In another example hereof, in the method of any of the previous or subsequent examples, the interlocking flange comprises a sloped face for sliding interaction with the flange of the distal tip, such that the flange of the distal tip is displaced from a longitudinal axis upon contacting the sloped face during the step of capturing the interlocking flange of the distal end of the distal capsule via the groove of the flange of the distal tip.

In another example hereof, the method of any of the previous or subsequent examples further includes securing the interlocking flange to the distal end of the distal capsule via an interlock collar.

In another example hereof, the method of any of the previous or subsequent examples further includes overlapping the proximal capsule with the distal capsule along a longitudinal axis, such that a proximal overlap collar on the distal end of the proximal capsule is received over a distal overlap collar on the proximal end of the distal capsule to create the overlap region, and disposing the removable sleeve over the overlap region to secure the proximal capsule to the distal capsule and form the outer sheath.

In another example hereof, in the method of any of the previous or subsequent examples, the step of attaching the device to the delivery system includes attaching the device to a distal end of a middle shaft having a proximal capture device disposed thereon.

In another example hereof, in the method of any of the previous or subsequent examples, removing the removable sleeve comprises tearing the removable sleeve along a perforation disposed along a length of the removable sleeve.

In another example hereof, a method of loading a device into a delivery system comprises attaching the device to the delivery system and radially compressing the device into an outer sheath of the delivery system. The outer sheath includes a proximal capsule having a distal end and a distal capsule having a proximal end and a distal end. During the radially compressing step, the distal end of the proximal capsule and the proximal end of the distal capsule overlap to form an overlap region. The method further includes securing a distal tip of the delivery system to the distal end of the distal capsule.

In another example hereof, the method of any of the previous or subsequent examples further includes interlocking the distal tip with an interlocking flange on the distal end of the distal capsule.

In another example hereof, the method of any of the previous or subsequent examples further includes capturing the interlocking flange of the distal end of the distal capsule via a groove of a flange of the distal tip.

In another example hereof, in the method of any of the previous or subsequent examples, the interlocking flange comprises a sloped face for sliding interaction with the flange of the distal tip, such that the flange of the distal tip is displaced from a longitudinal axis upon contacting the sloped face during the step of capturing the interlocking flange of the distal end of the distal capsule via the groove of the flange of the distal tip.

In another example hereof, the method of any of the previous or subsequent examples further includes securing the interlocking flange to the distal end of the distal capsule via an interlock collar.

In another example hereof, the method of any of the previous or subsequent examples further includes overlapping the proximal capsule with the distal capsule along a longitudinal axis, such that a proximal overlap collar on the distal end of the proximal capsule is received over a distal overlap collar on the proximal end of the distal capsule to create the overlap region, and disposing a removable sleeve over the overlap region to secure the proximal capsule to the distal capsule and form the outer sheath.

In another example hereof, the method of any of the previous or subsequent examples further includes removing the removable sleeve from the overlap region by pulling on a tab protrusion of the removable sleeve.

In another example hereof, in the method of any of the previous or subsequent examples, removing the removable sleeve comprises tearing the removable sleeve along a perforation disposed along a length of the removable sleeve.

In another example hereof, in the method of any of the previous or subsequent examples, the step of attaching the device to the delivery system includes attaching the device to a distal end of a middle shaft having a middle shaft capture device disposed thereon.

In another example hereof, a method of loading a device into a delivery system and delivering the device to a body lumen comprises overlapping a distal end of a proximal capsule with a proximal end of a distal capsule to form an overlap region, disposing a removable sleeve over the overlap region to secure the proximal capsule to the distal capsule, radially compressing the device into a distal opening of the distal capsule. Securing a distal tip to the distal end of the distal capsule, removing the removable sleeve from the overlap region removable sleeve, inserting the delivery system into the body lumen, and retracting the proximal capsule and distally advancing the distal capsule to separate the proximal capsule from the distal capsule such that the device to radially expands out of the proximal capsule and the distal capsule.

### BRIEF DESCRIPTION OF THE DRAWINGS

The accompanying figures, which are incorporated herein, form part of the specification and illustrate embodiments of a delivery system. Together with the description, the figures further explain the principles of and enable a person skilled in the relevant arts to make, use, and implant the prosthesis described herein. In the drawings, like reference numbers indicate identical or functionally similar elements.
FIG. 1A is a side view of an exemplary prosthetic valve device that may be utilized in embodiments hereof, wherein the prosthetic valve device is shown in its radially expanded or deployed configuration.
FIG. 1B is an end view of the prosthetic valve device of FIG. 1A.
FIG. 2 is a side view of a delivery system according to embodiments hereof.
FIG. 3 is a perspective view of a distal end of the delivery system of FIG. 2, showing a split capsule of the outer sheath having a proximal capsule and a distal capsule in an open configuration.
FIG. 4 is a perspective view the distal end of the delivery system of FIG. 3, wherein the proximal capsule and the distal capsule of the outer sheath are connected in a closed configuration, and a removable sleeve overlaps the proximal capsule and the distal capsule at an overlap region to secure the outer sheath in the closed configuration.
FIG. 5A is a perspective view of the distal end of the delivery system of FIG. 3 with the proximal capsule and the distal capsule of the outer sheath connected in the closed configuration, further showing the removable sleeve separated from the outer sheath.
FIG. 5B is a side cross-sectional view of the proximal capsule and the distal capsule of the outer sheath of FIG. 3 connected in the closed configuration and forming the overlap region.
FIG. 5C is a perspective view of the distal end of the delivery system of FIG. 3, showing the distal tip separated from a distal end of the distal capsule.
FIG. 5D is a perspective view of the distal end of the delivery system of FIG. 3, showing the distal tip separated from the distal end of the distal capsule.
FIG. 6A is a side perspective view of the delivery system of FIG. 2 with the prosthetic valve device of FIG. 1A disposed over a distal portion of the inner shaft, the proximal and distal capsules coupled together, and the outer sheath retracted.
FIG. 6B is a side perspective view of the delivery system of FIG. 2 with a proximal capture device of the delivery system engaged or attached to the prosthetic valve device of FIG. 1A.
FIG. 6C is a side perspective view of the delivery system of FIG. 2 and the prosthetic valve device of FIG. 1 with the middle shaft capture device of the delivery system engaged or attached to the prosthetic valve device and an inner shaft capture device engaged or attached to the prosthetic valve device.
FIG. 6D is a side perspective view of the delivery system of FIG. 2 and the prosthetic valve device of FIG. 1A as the prosthetic valve device is being collapsed and retracted into a loading funnel.
FIG. 6E is a side perspective view of the delivery system of FIG. 2 and the prosthetic valve device of FIG. 1A with the prosthetic valve device enclosed in the outer sheath of the delivery system in a radially compressed configuration.
FIG. 7A is a perspective view of the delivery system of FIGS. 2-3 with the prosthetic valve device enclosed in the outer sheath in a radially compressed configuration.
FIG. 7B is a perspective view of the delivery system of FIGS. 2-3 showing the removal of the removable sleeve from the outer sheath with the prosthetic valve device enclosed in the outer sheath in the radially compressed configuration.
FIG. 8 is a side elevational view of a distal end of the distal capsule of FIG. 3 configured for interlocking with the distal tip.
FIG. 9A is a side elevational view of the distal end of the distal capsule of FIG. 8 with the distal tip interlocked with the distal end of the distal capsule.
FIG. 9B is a close-up, cross-sectional view of the marked area of FIG. 9A showing the distal end of the distal capsule interlocked with the distal tip.
FIG. 10 is a flowchart illustrating a method of loading a device into a delivery system according to embodiments hereof.
FIG. 11A is a cross-sectional view of a treatment site and the delivery system of FIG. 2 inserted into the body lumen to deliver the prosthetic valve device therein.
FIG. 11B is a cross-sectional view of the treatment site of FIG. 11A with the proximal capsule retracted proximal of the first end of the prosthetic valve device such that the first end of the prosthetic valve device self-expands into the body lumen.
FIG. 11C is a cross-sectional view of the treatment site of FIG. 11A with the distal capsule extend distal of the second end of the prosthetic valve device such that the entire prosthetic valve service is fully expanded in the body lumen.

### DETAILED DESCRIPTION OF THE INVENTION

Specific embodiments of the present invention are now described with reference to the figures, wherein like reference numbers indicate identical or functionally similar elements. Unless otherwise indicated, the terms "distal" and "proximal", when used in the following description to refer to a shaft, a sheath, or a delivery device, are with respect to a position or direction relative to the treating clinician. Thus, "distal" and "distally" refer to positions distant from, or in a direction away from the treating clinician, and the terms "proximal" and "proximally" refer to positions near, or in a direction toward the treating clinician. In other words, when used with respect to a delivery device, the terms "distal" and "distally" refer to positions near or in a direction towards the tip of the delivery system, and the terms "proximal" and "proximally" refer to positions near or in a direction toward the handle of the delivery system. The terms "distal" and "proximal", when used in the following description to refer to a device to be implanted into a vessel, such as a prosthetic heart valve device, are used with reference to the direction of blood flow. Thus, "distal" and "distally" refer to positions in a downstream direction with respect to the direction of blood flow, and the terms "proximal" and "proximally" refer to positions in an upstream direction with respect to the direction of blood flow.

In addition, the term "self-expanding" is used in the following description with reference to one or more stent structures of the prostheses hereof and is intended to convey that the structures are shaped or formed from a material that can be provided with a mechanical memory to return the structure from a radially compressed or constricted radially compressed configuration to a radially expanded deployed configuration. Non-exhaustive illustrative self-expanding materials include stainless steel, a pseudo-elastic metal such as a nickel titanium alloy or nitinol, various polymers, or a so-called super alloy, which may have a base metal of nickel, cobalt, chromium, or other metal. Mechanical memory may be imparted to a wire or stent structure by thermal treatment to achieve a spring temper in stainless steel, for example, or to set a shape memory in a susceptible metal alloy, such as nitinol. Various polymers that can be made to have shape memory characteristics may also be suitable for use in embodiments hereof to include polymers such as polynorborene, trans-polyisoprene, styrene-butadiene, and polyurethane. As well poly L-D lactic copolymer, oligo caprylactone copolymer and poly cyclo-octine can be used separately or in conjunction with other shape memory polymers.

Embodiments hereof relate to a delivery system for delivery of a prosthetic valve device to a body lumen. The prosthetic valve device includes a first end and a second end. The delivery system includes an inner shaft and an outer sheath slidingly disposed over the inner shaft to hold the prosthetic valve device in a radially compressed configuration for delivery to a body lumen. The outer sheath comprises a proximal capsule and a distal capsule connectable to each other in open and closed configurations, wherein the open configuration comprises the proximal capsule and the distal capsule being separated from each other, and the closed configuration comprises the proximal capsule and the distal capsule being connected to each other. The proximal capsule comprises a distal end configured to overlap with a proximal end of the distal capsule when in the closed configuration, forming an overlap region. A removable sleeve is configured to be over the overlap region when the proximal capsule and the distal capsule are in the closed configuration to hold or secure the proximal capsule and distal capsule together.

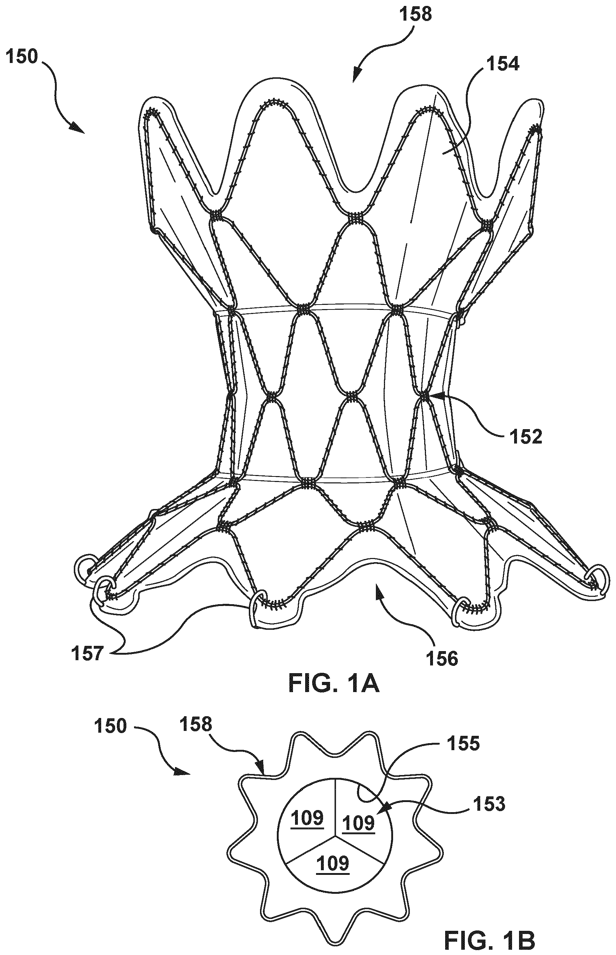

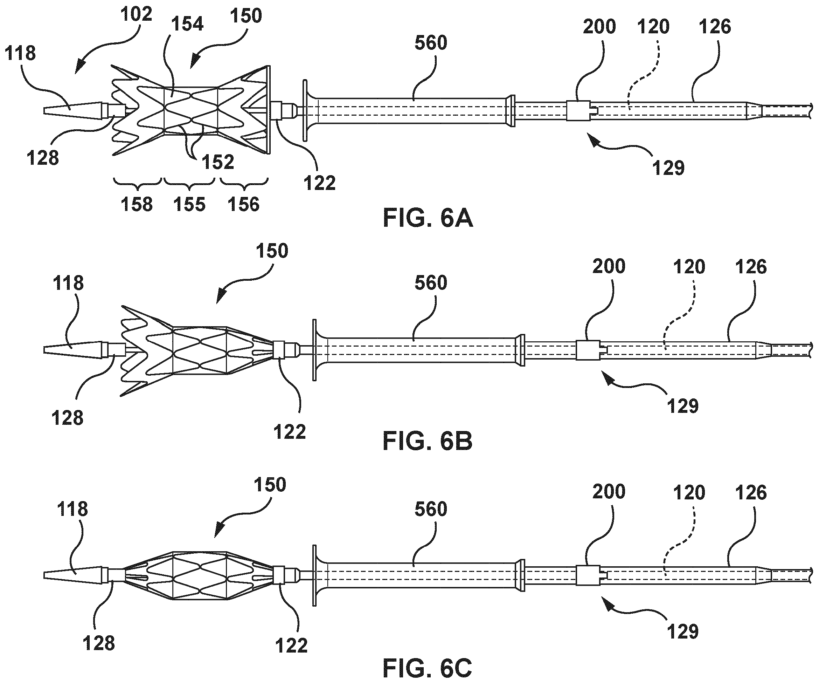

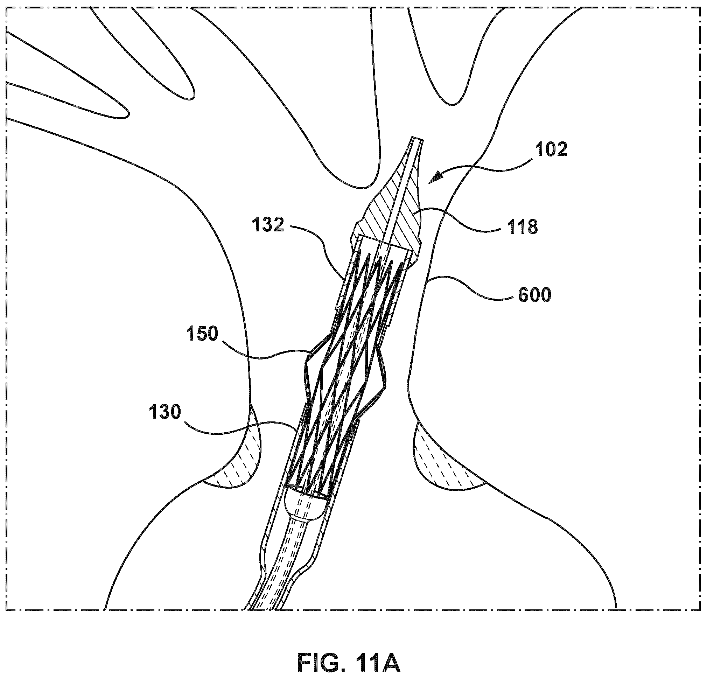

With reference to FIGS. 1A-1B, an exemplary prosthetic valve device 150 that may be used with the delivery systems described herein is shown. The prosthetic valve device 150 is merely an example, and it is understood that any number of alternate devices can be used with the delivery systems and methods described herein. Further, components may be added to or removed from the prosthetic valve device 150. The prosthetic valve device 150 includes a stent or expandable frame 152, a tubular graft or graft material 154, and a prosthetic valve component 153 disposed within and secured to the stent or frame 152 and/or graft material 154. More particularly, the valve component 153 is disposed within and secured to a midportion 155 of the graft material 154. A side view of the prosthetic valve device 150 is shown in FIG. 1A, while an end view of the prosthetic valve device 150 is shown in FIG. 1B. The stent or frame 152 is self-expanding or self-expandable and is configured to be radially compressed into a reduced-diameter crimped configuration for delivery within a vasculature and to return to a radially expanded or deployed configuration, as shown in FIGS. 6A-6E and 11A-11C and described in further detail herein. The prosthetic valve device 150 may include attachment members 157 at a first end 156 and/or a second end 158 for coupling the prosthetic valve device 150 to a delivery system 100. More particularly, the attachment members 157 are for coupling the prosthetic valve device 150 to a capture device or multiple capture devices 122, 128 to assist in loading the prosthetic valve device 150 on the delivery system 100.

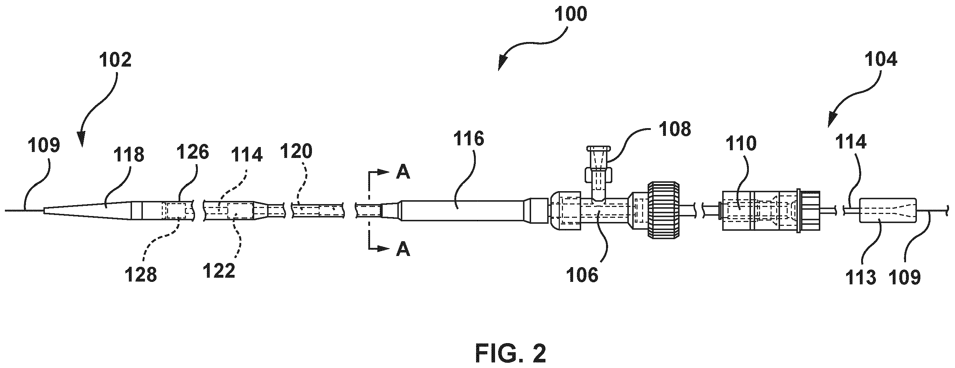

FIG. 2 depicts a side view of the delivery system 100 according to embodiments hereof. The delivery system 100 is configured to receive the prosthetic valve device 150 and deliver the prosthetic valve device 150 in the compressed or radially compressed configuration to treatment site, such as a body lumen, *in situ.* More particularly, the delivery system 100 includes a distal end 102 and a proximal end 104, wherein the proximal end 104 remains outside of the patient, and the distal end 102 is inserted into the patient and delivered intravascularly to a treatment area inside the body, such as a pulmonary valve. Other uses for the delivery system 100 in other areas of the body, however, are also contemplated. The proximal end 104 includes means for remotely controlling the distal end 102 of the delivery system 100, such as for the deployment or release of the prosthetic valve device 150 from the delivery system 100 *in situ.*

The proximal end 104 of the delivery system 100 may include a first homeostasis valve 106, a side access port 108, a second homeostasis valve 110, and/or a handle 113. The first homeostasis valve 106 and the second homeostasis valve 110 grip or form a fluid seal around an outer surface of the delivery system 100 to prevent blood or other fluids from leaking out of the delivery system 100 at the proximal end 104 or entry site into a patient, and are further configured to allow for the passage of wires, devices, or fluids therethrough. The first homeostasis valve 106 and second homeostasis valve 110 may further control the components on the distal end 102 of the delivery system 100, as further described herein. The side access port 108 provides a means for injecting contrast media or saline into the delivery system 100. The proximal end 104 of the delivery system 100 may further include retraction and/or advancement mechanisms for retracting and/or advancing shafts of the delivery system 100. The retraction and/or advancement mechanisms may be knobs, rotatable members, axially-slidable levers, rotatable rack and pinion gears, or other known mechanisms. Other alternative or additional components of the proximal end 104 of the delivery system 100 are also contemplated by the invention.

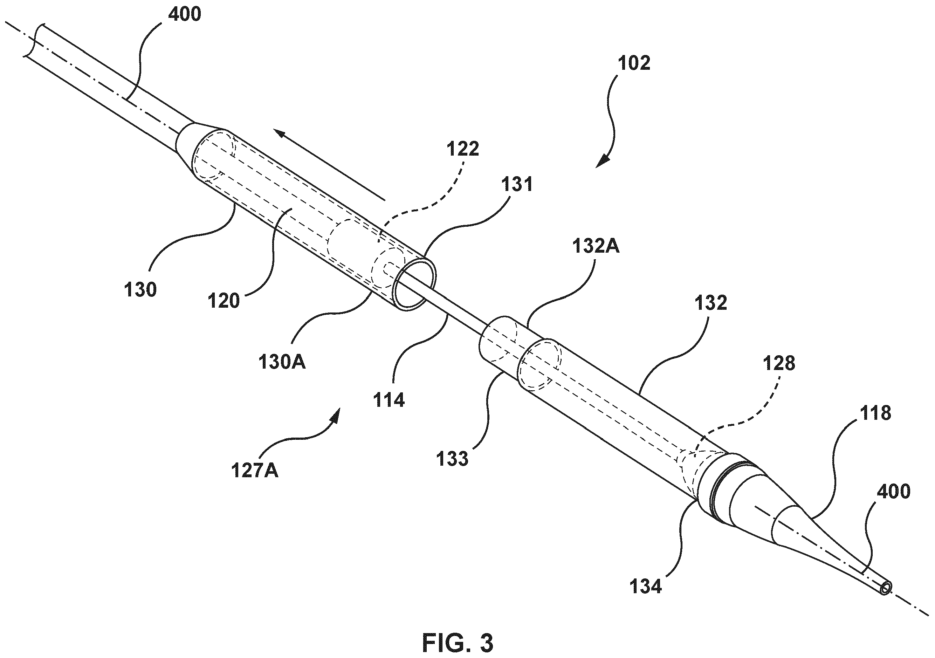

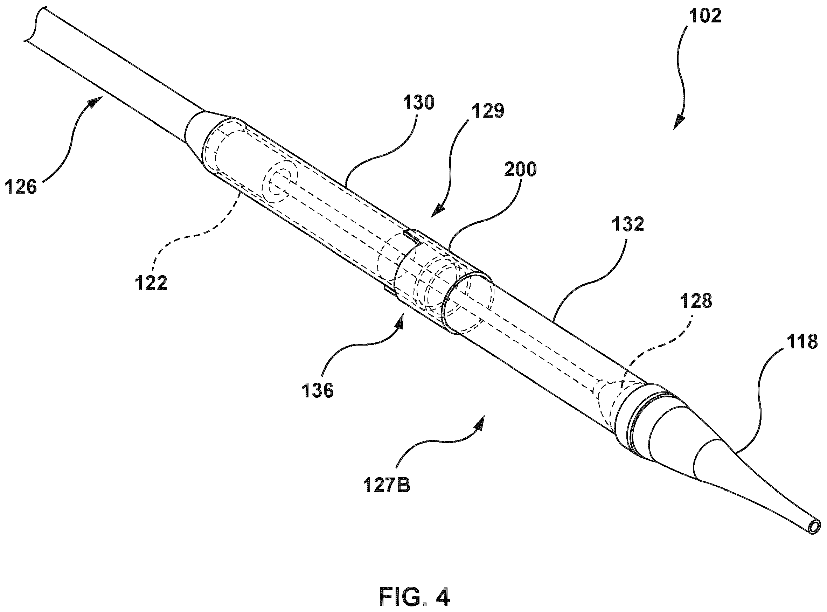

The distal end 102 of the delivery system 100 is shown in an assembled configuration in FIGS. 2-4 with greater detail, without a prosthetic valve device 150 attached to or loaded therein. The distal end 102 of the delivery system 100 includes an inner shaft 114, a middle shaft 120 slidingly disposed over the inner shaft 114, and an outer sheath 126 slidingly disposed over the middle shaft 120 and configured to hold the prosthetic valve device 150 in a radially compressed configuration during delivery to a body lumen. A first or distal capture device 128 may be disposed on the distal end of the inner shaft 114, the distal capture device 128 being configured to connect with the prosthetic valve device 150 for loading into the delivery system 100. More particularly, the distal capture device 128 is configured to interact with or connect to the attachment members 157 located on the first end 156 and/or the second end 158 of the prosthetic valve device. A second or proximal capture device 122 may be disposed on a distal end of the middle shaft 120, the proximal capture device 122 being configured to connect with the prosthetic valve device 150 for loading into the delivery system 100. More particularly, the proximal capture device 122 is configured to interact with or connect to the attachment members 157 located on the first end 156 and/or the second end 158 of the prosthetic valve device 150. FIG. 2 shows the delivery system 100 with both the proximal and distal capture devices 122, 128. FIGS. 3 and 4 show the delivery system 100 with only the proximal capture device 122. Either embodiment may be utilized. In other embodiments (not shown), the delivery system 100 may include only the distal capture device 128. The proximal and distal capture devices 122, 128 may be coils such as those described in U.S. Patent Application Publication No. 2021/0346158 A1. However, this is not meant to be limiting, and other capture devices may also be used.

The delivery system 100 may also include an outer sleeve 116 disposed over a proximal portion of the outer sheath 126 to prevent blood or other fluids from leaking back around the delivery system 100. A tapered distal tip 118 is attached to a distal end of the inner shaft 114 and is configured or shaped to ease passage of the delivery system 100 through the vasculature. The inner shaft 114 extends along the entire length of the delivery system 100 (i.e. along its longitudinal axis 400) and is configured to be tracked over a guidewire 109, as shown in FIG. 2. The inner shaft 114 is attached to a handle 113 at its proximal end. Inner shaft 114 may be independently advanced or retracted through other components of the delivery system 100 by moving the handle 113, and inner shaft 114 may be rotated about the longitudinal axis 400 by rotation of or turning of the handle 113.

FIGS. 3-5D illustrate the distal end 102 of the delivery system 100 according to embodiments hereof. A distal portion of the outer sheath 126 includes a split capsule 129 comprising a proximal capsule 130 and a distal capsule 132. The proximal and distal capsules 130, 132 may be tubular bodies that may be joined together for loading the prosthetic valve device 150 therein and delivering the prosthetic valve device 150 to a treatment site, and may be separated during deployment of the prosthetic valve device 150, as described below. In particular, the proximal capsule 130 includes a distal end 131 configured to connect with a proximal end 133 of the distal capsule 132. In embodiments, the distal end 131 of the proximal capsule 130 includes a proximal overlap collar 130A extending distally and configured to overlap with a distal overlap collar 132A included on the proximal end 133 of the distal capsule 132 and extending proximally. The proximal overlap collar 130A and/or distal overlap collar 132A may be tubular bodies. In the embodiment shown, the proximal overlap collar 130A and the distal overlap collar 132A are cylindrical, but in other embodiments may be other shapes that allow for a connecting/mating/overlapping relationship, such as, but not limited to, oval, oblong, square, pentagonal, hexagonal, heptagonal, octagonal, or any other type of shape capable of achieving a mating/overlapping relationship with one another. In other embodiments, the proximal capsule 130 and distal capsule 132 may abut each other. In such an embodiments, the proximal overlap collar 130A and the distal overlap collar 132A are not necessary.

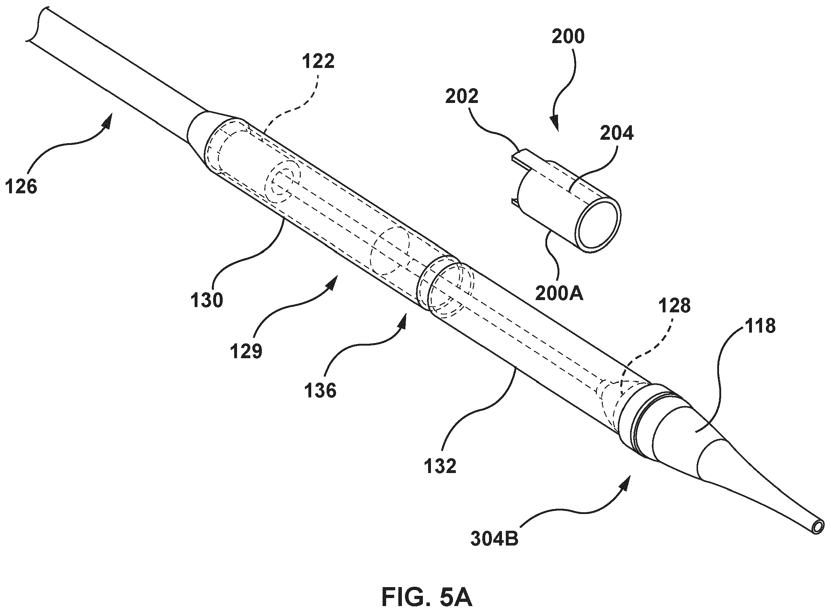

In the embodiment shown, the proximal overlap collar 130A is received over the distal overlap collar 132A to form an overlap region 136. However, this is not meant to be limiting, and in other embodiments, the distal overlap collar 132A may be received over the proximal overlap collar 130A to form the overlap region 136. The proximal capsule 130 and the distal capsule 132 enable the split capsule 129 of the outer sheath 126 to transition between an open configuration 127A, where the proximal capsule 130 and the distal capsule 132 are disconnected/split/separated from each other (as shown in FIG. 3), and a closed configuration 127B where the proximal capsule 130 and the distal capsule 132 are connected to each other or overlapped at their distal end 131 and proximal end 133, respectively (as shown in FIG. 4). When in the closed configuration 127B, a removable sleeve 200, as shown in FIG. 5A, may be disposed over the overlap region 136 of the split capsule 129 to secure the proximal capsule 130 to the distal capsule 132 to prevent separation, breakage, or bending of the split capsule 129 during loading of the prosthetic valve device 150 into the delivery system 100 as further described below. In embodiments without an overlap region 136, the removable sleeve 200 may overlap the distal end of the proximal capsule 130 and the proximal end of the distal capsule 132, thereby joining the proximal and distal capsules 130, 132 together.

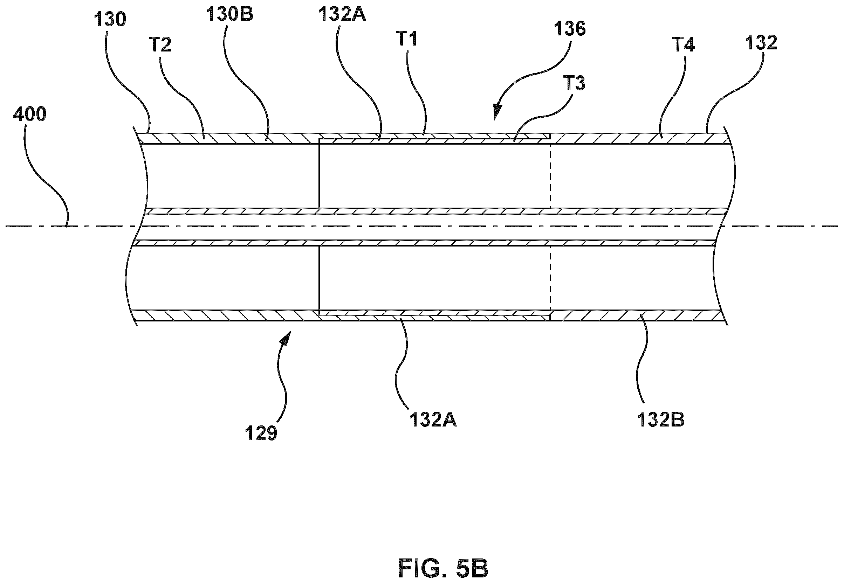

As shown in FIG. 5B. the proximal collar 130A may be a reduced thickness portion of the proximal capsule 130 such that a first thickness T1 of the wall of the proximal collar 130A is smaller than a second thickness T2 of the wall of a body portion 130B of the proximal capsule 130 proximal of the proximal collar 130A. Similarly, the distal collar 132A may be a reduced thickness portion of the distal capsule 132 such that a third thickness T3 of the wall of the distal collar 132A is smaller than a fourth thickness T4 of the wall of a body portion 132B of the distal capsule 132 distal of the distal collar 132A.In an embodiment, the combined thicknesses T1, T3 of the wall of the proximal collar 130A and the distal collar 132A may be approximately equivalent to the thickness T2 or T4 of the body portions 130B, 132B of the proximal capsule 130 or the distal capsule 132, respectively. Accordingly, a profile (*i.e.,* outer diameter) of the overlap region 136 is no greater than a profile of the body portion 130B of the proximal capsule 130 and/or the body portion 132B of the distal capsule 132.

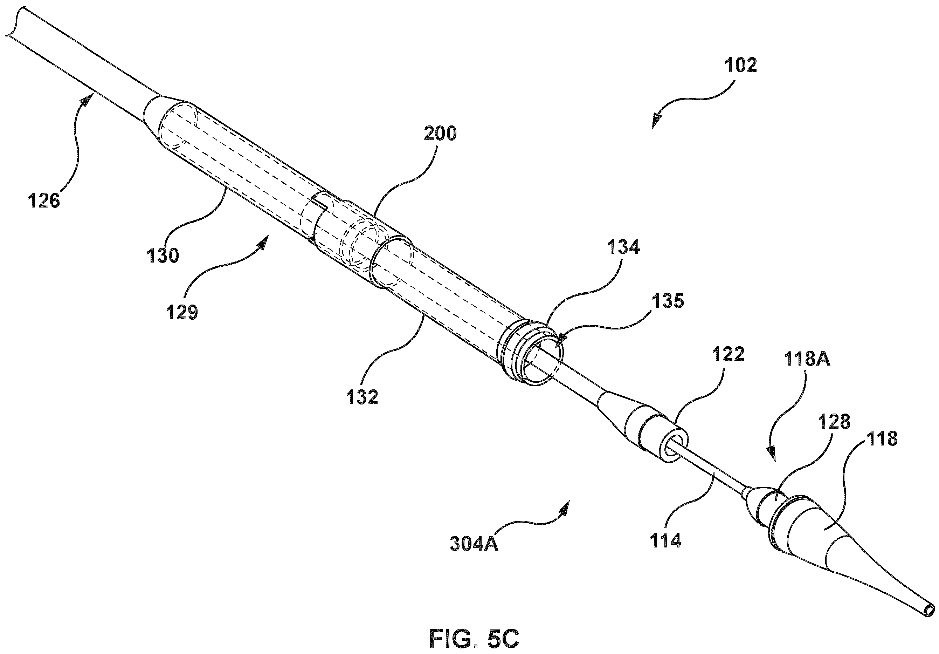

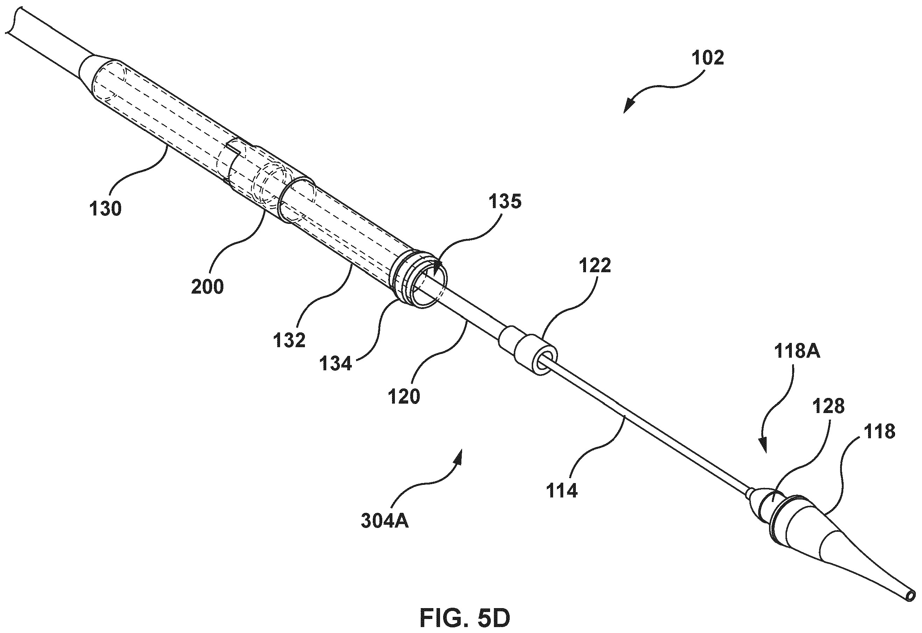

As noted above, with the proximal capsule 130 and the distal capsule 132 in the closed configuration 127B, the removable sleeve 200 may be disposed over the overlap region 136 to secure the proximal capsule 130 and the distal capsule 132 together, as illustrated in FIGS. 4, 5C, and 5D. More particularly, disposing the removable sleeve 200 on the overlap region 136 of the split capsule 129 of the outer sheath 126 prevents unwanted bending, breakage, or separation of the proximal capsule 130 and the distal capsule 132 during loading of the prosthetic valve device 150 into the delivery system 100. Use of the removable sleeve 200 with the delivery system 100 described herein also improves alignment between the proximal capsule 130 and the distal capsule 132, and eliminates the need for an outer sheath with a thicker wall/body, and/or a higher stiffness, which would be required using a proximal capsule and distal capsule without the removable sleeve 200. Use of the removable sleeve 200 also enables a thinner capsule wall as compared to delivery systems that utilize a single outer sheath (i.e., a single, non-split capsule). This increased wall thickness and/or higher stiffness are both undesirable for tracking and anatomical compatibility. Thus, use of the removable sleeve 200 with the delivery system 100 described herein ensures use of an outer sheath profile and flexibility that does not compromise performance during the steps of installation of the prosthetic valve device 150 in the body lumen.

As shown in FIG. 5A, the removable sleeve 200 is a tubular sleeve that includes a body 200A and at least one tab protrusion 202 extending from the body 200A. The tab protrusion 202 facilitates end user interactions, such as grabbing, pulling, or removing the removable sleeve 200 from the capsule 129 of the outer sheath 126. More specifically, the at least one tab protrusion 202 may be a square, rectangular, circular, oblong, or any other appropriately shaped protrusion extending from the body 200A of the removable sleeve 200. In embodiments, at least one tab protrusion may be bumps, extensions, or other inconsistencies formed on the body 200A of the removable sleeve 200 that facilitate grabbing/interacting by the end user. In embodiments, a plurality of tab protrusions 202 may extend from the body 200A to provide an end user with easier removal of the removable sleeve 200 from the delivery system 100. In particular, in the embodiment shown the removable sleeve includes two tab protrusions 202 disposed opposite each other around a circumference of the body 200A. The removable sleeve 200 may further include at least one perforation 204 along the length of the body 200A such that the body 200A is tearable along the perforation 204 to assist in removal of the removable sleeve 200 from the split capsule 129 of the outer sheath 126 In an embodiment, the removable sleeve 200 fully envelops the overlap region 136 of the split capsule 129, such that the removable sleeve 200 covers the entire length and circumference the overlap region 136. Alternatively, the removable sleeve 200 may only cover a portion of the length of the overlap region 136. Further, in embodiments with abutting proximal and distal capsules 130, 132, the removable sleeve 200 covers the abutting region of the proximal and distal capsules 130, 132. The removable sleeve 200 may be formed of elastomeric or other rubberlike polymer materials to enable loading onto the capsule and achieving a tight fit in the overlapped region. The material selected could range in durometer from 30A - 55D shore hardness, or in other instances may consist of a mix of elastomeric alongside a more rigid polymer to provide the tear release mechanism, for example. Further, the body 200A may have a length of about 5 to about 40 mm.

FIGS. 5C and 5D illustrate the distal tip 118 of the delivery system 100 according to embodiments hereof. In particular, a distal end of the inner shaft 114 is coupled to the distal tip 118. Translating the inner shaft 114 proximally or distally along the longitudinal axis 400 of the delivery system 100 results in the distal tip 118 translating between a loading or open configuration 304A (as shown in FIGS. 5C and 5D), and a connected or closed configuration 304B (as shown in FIG. 5A). In the loading or open configuration 304A, the distal tip 118 is separated from the distal end of the distal capsule 132. In the closed configuration 304B, the distal tip 118 is connected to or adjacent to the distal end of outer sheath 126, and more specifically to the distal capsule 132 of the outer sheath 126, to secure the loaded prosthetic valve device 150 into the delivery system 100. More particularly, a proximal end 118A of the distal tip 118 is configured to interlock with a distal end 134 of the distal capsule 132 when in the closed configuration 304B.

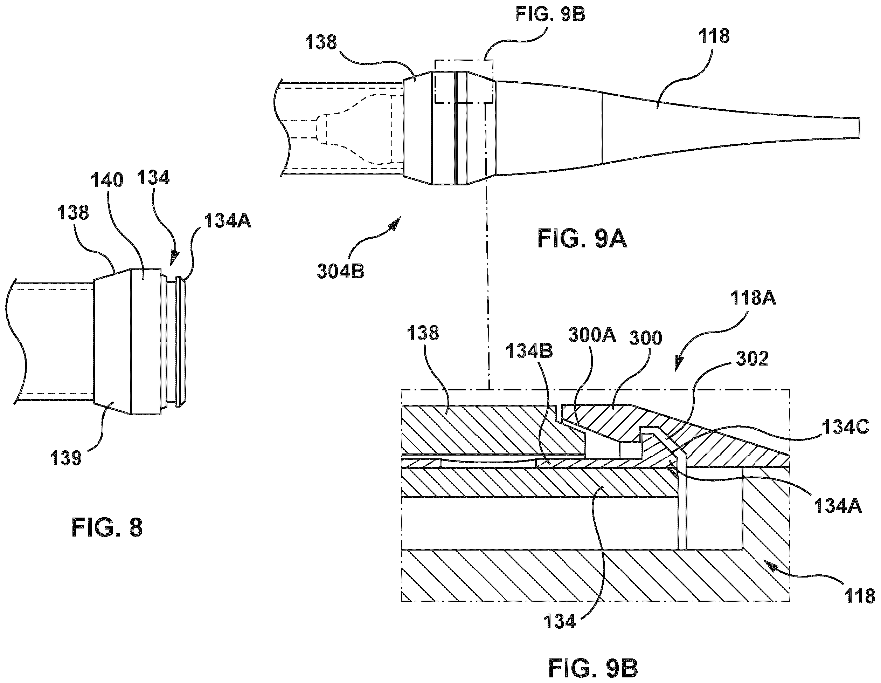

In an embodiment, as shown in FIGS. 8-9B the proximal end 118A of the distal tip 118 includes a flange 300 having a groove 302 therein for receiving a corresponding feature of the distal end 134 of the distal capsule 132. For example, in an embodiment, the distal end 134 of the distal capsule 132 comprises an interlocking flange 134A extending radially outward from the distal end 134 of the distal capsule 132. In an embodiment, the interlocking flange 134A extends around the entire circumference of the distal end 134 of distal capsule 132, but this is not meant to be limiting. Further, the interlocking flange 134A may extend approximately perpendicular to the longitudinal axis 400 as illustrated in FIGS. 8 and 9B. The interlocking flange 134A may be a separate element coupled to the distal end 134 of the distal capsule 132, as shown in FIG. 9B, or it may be formed integrally with the distal end 134 of the distal capsule 132. The interlocking flange 134A at the distal end 134 of the distal capsule 132 may include a sloped or angled face 134C for sliding interaction with a corresponding sloped face 300A of the flange 300 of the distal tip 118, such that the flange 300 of the distal tip 118 is displaced from the longitudinal axis 400 upon contacting the sloped face 134C of the interlocking flange 134A during interlocking of the distal tip 118 with the distal end 134 of the distal capsule 132. In embodiments, the distal tip 118 may comprise a material having a durometer range between 30A - 65D shore hardness, so as to allow for the displacement of the flange 300 of the distal tip 118 from the longitudinal axis 400 upon contacting the sloped face 134C of the interlocking flange 134A. In other words, the material of the distal tip 118 enables the flange 300 to expand or bend outwards as the sloped face 300A of the flange 300 slides against the sloped face 134C of the interlocking flange 134A. In the embodiment shown the sloped face 134C of the interlocking flange 134A tapers in a distal direction such that the interlocking flange 134A at a distal end of the sloped face 134C has a smaller diameter than the interlocking flange 134A at a proximal end of the sloped face 134C. Similarly, the sloped face 300A of the flange 300 of the distal tip 118 tapers in a proximal direction such that the flange 300 at a distal end of the sloped face 300A has a larger diameter than the flange 300 at a proximal end of the sloped face 300A. In embodiments, the distal end 134 of the distal capsule 132 may comprise any suitable protrusion, flange, projection, or feature designed to mate with, connect to, interlock with, or otherwise secure itself to the proximal end 118A of the distal tip 118, which may also comprise any cooperative protrusion, flange, projection, or feature design for said mating, connecting, interlocking, or securing procedure. Interlocking the distal tip 118 and the distal end 134 of the distal capsule 132 ensures integrity and stability of the delivery system 100 once the prosthetic valve device 150 is loaded and the removable sleeve 200 is removed from the outer sheath 126. In embodiments, the connection between the interlocking flange 134A and the flange 300 of the distal tip 118 occurs within a 5-millimeter region, which maintains delivery system 100 performance for tracking, and ensures the stiffness of the distal tip 118 during insertion procedures. Furthermore, the connection between the distal tip 118 and the distal end 134 of the distal capsule 132 ensures the distal end 102 of the delivery system 100 moves as one component during the steps of deploying the prosthetic valve device 150 from the distal capsule 132. In embodiments, the interlocking flange 134A comprises a metal or a rigid polymer, or any other suitable material composition with enough structural integrity to secure the distal tip 118 thereto without concern of damage, warping, improper interlock, or complete break-off.

The distal end 134 of the distal capsule 132 may also include an interlock collar 138 disposed on the distal end 134 and enveloping the outer surface of the distal end 134. In embodiments, the interlock collar 138 integrates or secures the interlocking flange 134A to the distal end 134 of the distal capsule 132. In the embodiment shown, the interlock collar 138 radially compresses the interlocking flange 134A to the outer surface of the distal capsule 132. In the embodiment shown, the interlock collar 138 extends around the entire circumference of a longitudinal base 134B of the interlocking flange 134A and the entire circumference of the distal end 134 of the distal capsule 132. In the embodiment shown, the interlock collar 138 includes a proximal tapered portion 139 and a distal cylindrical portion 140, as shown in FIG. 8. The distal cylindrical portion 140 aligns with the distal tip 118 such that an outer diameter of the distal cylindrical portion 140 is about the same as an outer diameter of a proximal end of the distal tip 118, to providing a smooth transition therebetween. The proximal tapered portion 139 tapers in the proximal direction such that an outer diameter at the distal end of the proximal tapered portion 139 matches the outer diameter of the distal cylindrical portion 140 and is larger than an outer diameter at the proximal end of the proximal tapered portion 139. In embodiments, the interlock collar 138 comprises a polymer, such as, but not limited to PEEK, Pebax, polyimide, polyamide, polycarbonate, polyethylene, polyurehane or metals such as stainless steel, cobalt chrome, or any other suitable material with sufficient structural integrity to ensure that the interlocking flange 134A is properly secured to the distal end 134 without concern of displacement of the interlocking flange 134A during loading the prosthetic valve device and delivering and deploying the prosthetic valve device 150 to the treatment site.

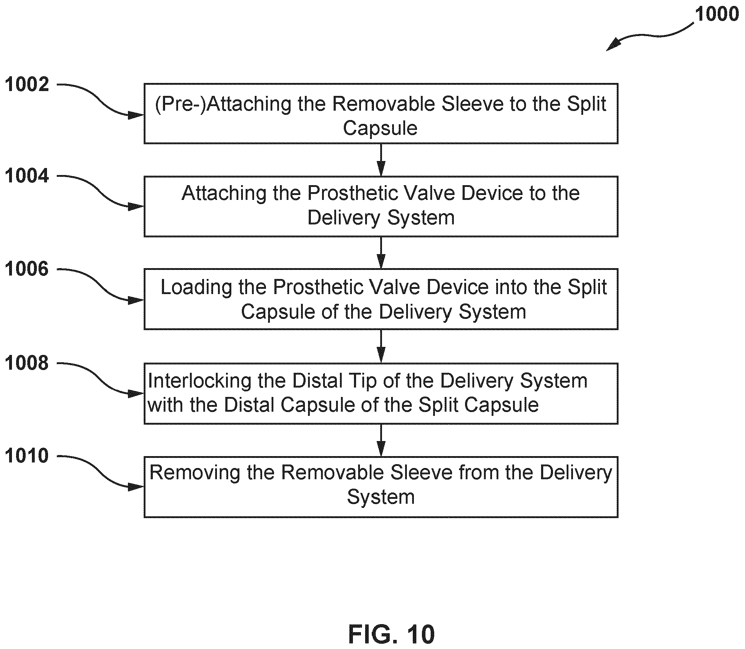

FIGS. 6A-6E and 10 illustrate a method 1000 of loading the prosthetic valve device 150 onto the delivery system 100, which is done outside of the patient's body prior to insertion of the delivery system 100 into a body lumen, such as a blood vessel (e.g., vein or artery). In a step 1002 of the method 1000, the removable sleeve 200 is attached to the delivery system 100 with the split capsule 129 of the outer sheath 126 in the closed configuration. More particularly, the removable sleeve 200 is disposed over the overlap region 136 of the split capsule 129 with the split capsule 129 in the closed configuration. In embodiments of the step 1002 of the method 1000, attaching the removable sleeve 200 to the delivery system 100 may be performed by the manufacturer. In other words, the delivery system 100 may be provided with the proximal and distal capsules 130, 132 coupled to each other via the removable sleeve 200, so as simplify the loading method for the end user. In other words, the end user may not be required to attach the removable sleeve 200 to the delivery system 100 to couple the proximal and distal capsules 130, 132 to each other, as the removable sleeve 200 may be attached to the delivery system 100 coupling the proximal and distal capsules 130, 132 together prior to being received by and interacted with the end user while engaging in steps 1004-1010 of the method 1000.

In a step 1004 of the method 1000, the prosthetic valve device 150 is attached to the delivery system 100. In particular, FIG. 6A is a side perspective view of the delivery system 100 and illustrates the prosthetic valve device 150 surrounding the distal end 102 of the delivery system 100 prior to loading or attaching the prosthetic valve device 150 onto the delivery system 100. The prosthetic valve device 150 is shown in its expanded configuration in FIG. 6A and is not yet attached to the delivery system 100. As part of the step 1004, the distal end 102 of the delivery system 100, and specifically the inner shaft 114 and the distal tip 118, is extended through a central lumen of the prosthetic valve device 150, with the split capsule 129 retracted relative to the distal tip 118. The distal tip 118 is extended through the prosthetic valve device 150 until the first end 156 of the prosthetic valve device 150 is adjacent to the proximal capture device 122 of the delivery system 100. As also shown in FIG. 6A, the split capsule 129 is in the closed configuration with the removable sleeve 200 disposed over the overlap region 136 of the split capsule 129. In another step of the method, as shown FIG. 6B, the first end 156 of the prosthetic valve device 150 is coupled to the proximal capture device 122. Also, as part of the step 1004, as shown in FIG. 6C, the second end 158 of prosthetic valve device 150 is coupled to the distal capture device 128. As noted above, in embodiments, the delivery system 100 may include only the proximal capture device 122 or only the distal capture device 128, in which case, only the step shown in FIG. 6B or FIG. 6C, respectively, would be necessary. Further, the steps of FIG. 6B and 6C may be performed in reverse order. In other embodiments, the delivery system 100 may not include any capture device, in which case, the steps of FIGS. 6B and 6C need not be performed.

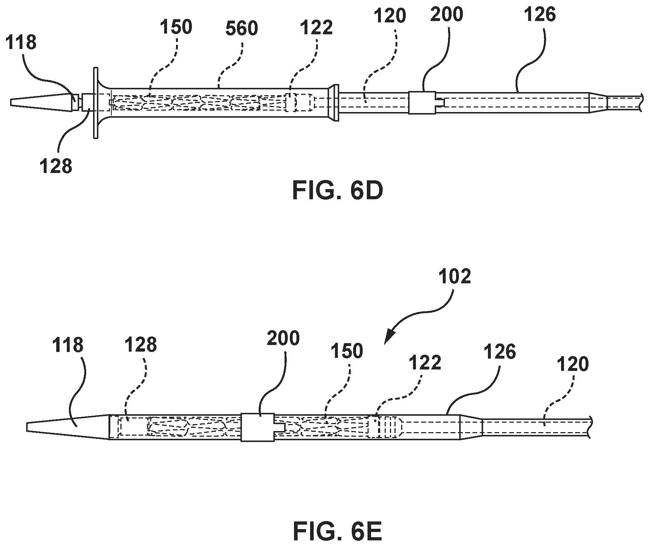

In a step 1006 of the method 1000, the prosthetic valve device 150 is loaded into the split capsule 129. In particular, as shown in FIG. 6D, the prosthetic valve device 150 may be pulled or retracted into the delivery system 100 through a loading funnel 560 used to radially compress the prosthetic valve device 150 and load the prosthetic valve device 150 into the split capsule 129 of the delivery system 100. In other embodiments, the split capsule 129 with the loading funnel 560 disposed thereover is distally advanced to radially compress the prosthetic valve device 150. More particularly, the prosthetic valve device 150 may be pulled or retracted the loading funnel 560 and through a distal end opening 135 at the distal end 134 of the distal capsule 132 (as shown in FIG. 5D). As the prosthetic valve device 150 is loaded into the split capsule 129 of the outer sheath 126, the split capsule 129 remains in the closed configuration with the removable sleeve 200 disposed over the overlap region 136. As further shown in FIG. 6E, the prosthetic valve device 150 is further retracted proximally into the split capsule 129 of the outer sheath 126 of the delivery system 100, until the collapsed prosthetic valve device 150 is completely disposed within the split capsule 129.

In a step 1008 of the method 1000, the distal tip 118 is translated proximally, such as by pulling the inner shaft 114, until the distal tip 118 attaches, interlocks, or abuts the distal end 134 of the distal capsule 132. In particular, in the embodiment shown, the distal tip 118 is translated proximally until the interlocking flange 134A of the distal capsule 132 interlocks with the groove 302 of the flange 300 of the distal tip 118, as shown in FIG. 9B.

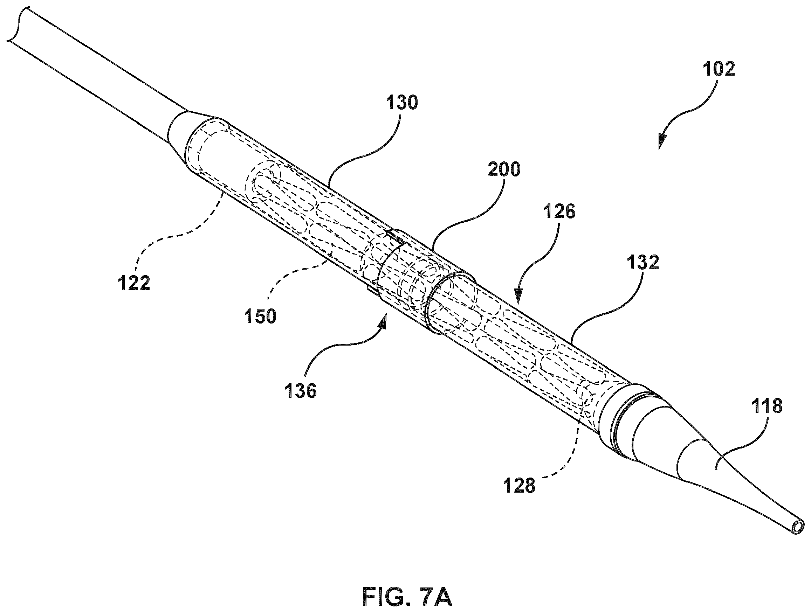

FIG. 7A illustrates the distal end 102 of the delivery system 100 with the prosthetic valve device 150 fully loaded into the delivery system 100, or more particularly disposed within the split capsule 129, after completion of the loading steps previously described herein. The split capsule 129 is in the closed configuration with the removable sleeve 200 disposed over the overlap region 136 to provide stability and ensure that the proximal capsule 130 and the distal capsule 132 stay connected.

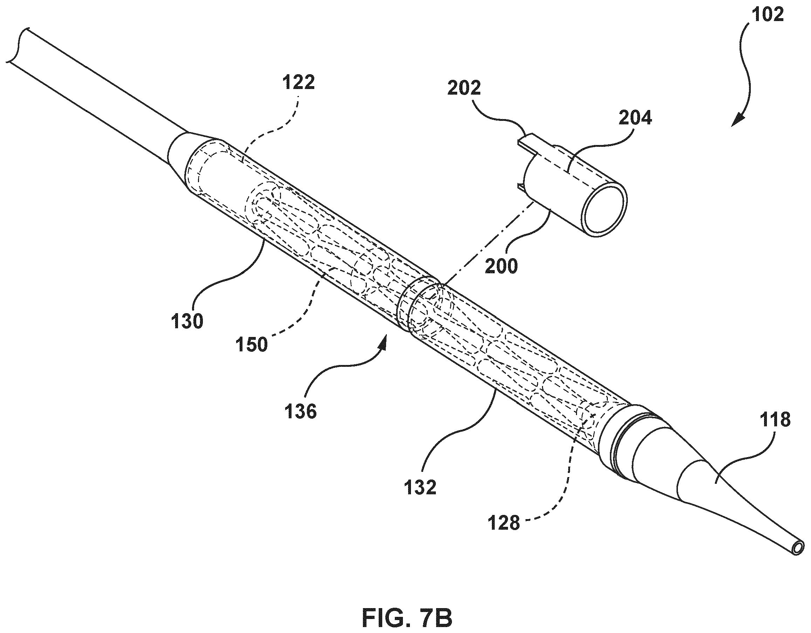

After the completion of the loading steps previously described herein, in a step 1010 of the method 1000, the removable sleeve 200 may be removed from the split capsule 129 of the outer sheath 126, as illustrated in FIG. 7B, while the prosthetic valve device 150 remains fully loaded into the delivery system 100. Removal of the removable sleeve 200 from the outer sheath 126 may include grasping the at least one tab protrusion 202 and pulling, ripping, tearing, or otherwise applying a force in a direction moving away from the delivery system 100 so as to cause detachment of the removable sleeve 200. In embodiments, pulling the at least one tab protrusion 202 may result in an assisted tear along the at least one perforation 204 disposed along the length of the body 200A of the removable sleeve 200, as previously described herein. With the prosthetic valve device 150 loaded into the split capsule 129 of the delivery system 100 with the split capsule in a closed configuration, and the removable sleeve removed from the delivery system 100, the delivery system 100 with the prosthetic valve device 150 loaded therein is ready to be inserted into a body lumen or treatment site 600, such as a blood vessel.

FIGS. 11A-11C illustrate a method of delivering the prosthetic valve device 150 into the body lumen or treatment site 600 after loading the prosthetic valve device 150 into the delivery system 100 using the methods described herein. The distal end 102 of the delivery system 100 is inserted into a body lumen, such as a blood vessel, by techniques known to those skilled in the art, and delivered to a treatment site. FIG. 11A shows the distal end 102 of the delivery system 100 at a treatment site 600 Upon reaching the treatment site 600, the proximal capsule 130 and the distal capsule 132 of the outer sheath 126 are separated from each other. In particular, the proximal capsule 130 may be retracted proximally and/or the distal capsule 132 may be advanced distally. Such a separation enables a middle portion of the prosthetic valve device 150 to partially radially expand, as shown in FIG. 11A, as the middle portion of the prosthetic valve device 150 is no longer restrained within the split capsule. As can be seen in FIG. 11A, the first and second ends 156, 158 of the prosthetic valve device 150 remain in the proximal and distal capsules 130, 132, respectively. At this step, the location of the prosthetic valve device 150 can be checked and adjusted if needed. In particular, if the location needs to be adjusted, the proximal capsule 130 can be advanced distally to recapture the middle portion of the prosthetic valve device 150, and the delivery system 100 can be moved to adjust the location.

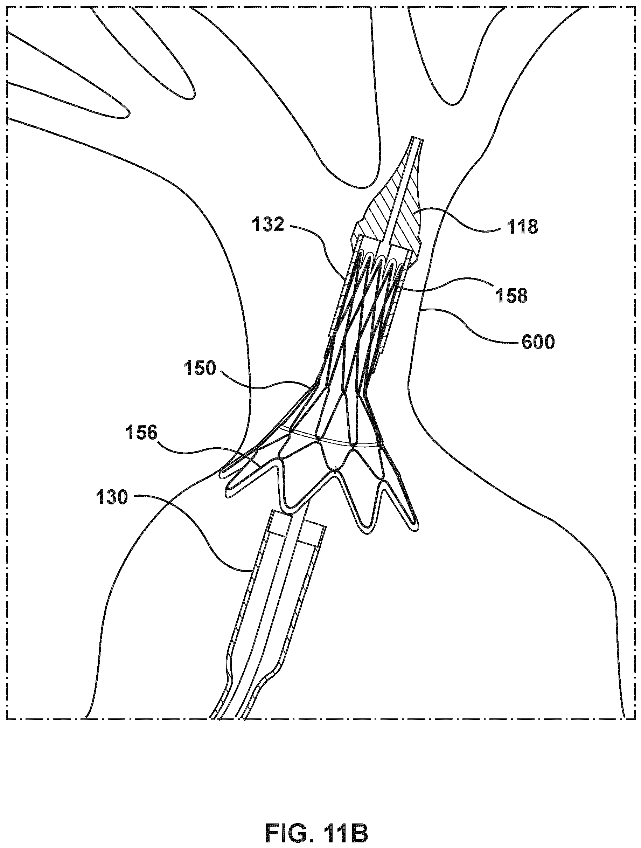

In another step, once the proper alignment is achieved, the proximal capsule 130 may be further retracted proximally away from the distal capsule 132 such that the proximal capsule 130 is disposed proximal of the first end 156 of the prosthetic valve device 150, thereby enabling the first end 156 of the prosthetic valve device 150 to release from the delivery system 100 and fully expand within the body lumen at the treatment site 600, as shown in FIG. 11B. As this first end 156 expands, it anchors to the body lumen at the treatment site.

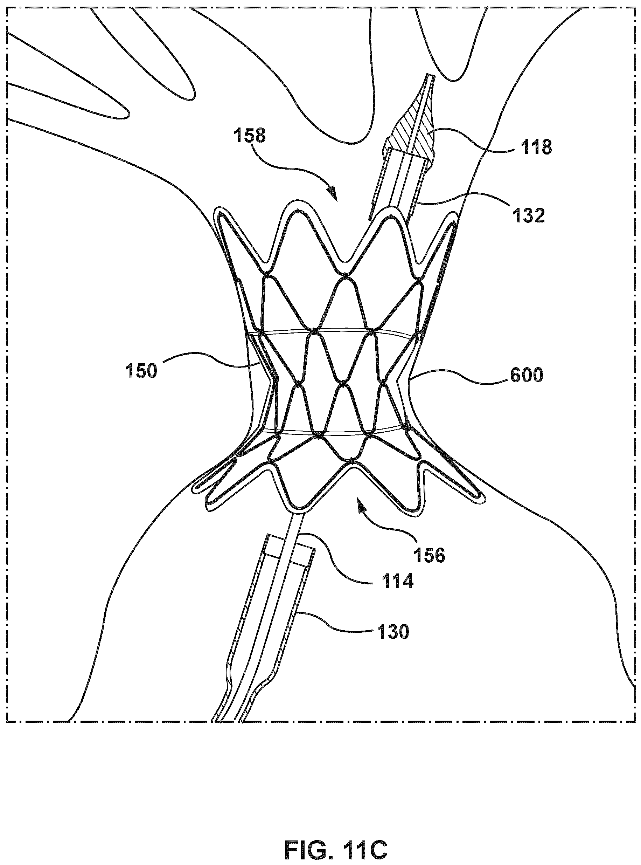

With the first end 156 of the prosthetic valve device 150 radially expanded at anchored at the treatment site 600, the distal capsule 132 may be advanced distally past the second end 158 of the prosthetic valve device 150 by pushing the inner shaft 114 distally. With the distal capsule 132 advanced past the second end 158 of the prosthetic valve device, the second end 158 of the prosthetic valve device 150 is released from the delivery system 100 such that it can radially expand within the treatment site 600, as shown in FIG. 11C.

Once prosthetic valve device 150 is fully expanded and released, the proximal and distal capsules 130, 132 may be brought back together, and the distal end 102 of the delivery system 100 may be retracted through the center of the prosthetic valve device 150 and removed from the patient.

In embodiments, the treatment site 600 may be different locations within body lumens, such as blood vessels. For example, and not by way of limitation, the treatment site 600 may a native right ventricular outflow tract (RVOT), a native inferior vena cava (IVC), or a native superior vena cava (SVC).

Further disclosed herein is a delivery system for delivering a device to a body lumen. The delivery system includes an inner shaft coupled to a distal tip and an outer sheath slidingly disposed over the inner shaft. The outer sheath is configured to hold the device in a radially compressed configuration. The outer sheath includes a proximal capsule and a distal capsule. The delivery system further includes a removable sleeve configured to couple the proximal capsule to the distal capsule during loading of the device into the delivery system. A distal end of the distal capsule is configured to interlock with the distal tip after the device is loaded into the delivery system.

The foregoing description has been presented for purposes of illustration and enablement and is not intended to be exhaustive or to limit the invention to the precise form disclosed. Other modifications and variations are possible in light of the above teachings. The embodiments and examples were chosen and described in order to best explain the principles of the invention and its practical application and to thereby enable others skilled in the art to best utilize the invention in various embodiments and various modifications as are suited to the particular use contemplated.

## Claims

1. A delivery system (100) for delivering a device (150) to a body lumen, the delivery system comprising:
an inner shaft (114) coupled to a distal tip (118);
an outer sheath (126) slidingly disposed over the inner shaft configured to hold the device in a radially compressed configuration, the outer sheath having a proximal capsule (130) with a distal end (131), and a distal capsule (132) with a proximal end (133), the distal end of the proximal capsule configured to overlap with the proximal end of the distal capsule to form an overlap region (136); and
a removable sleeve (200) configured to couple the proximal capsule to the distal capsule at the overlap region.

2. The delivery system of claim 1, wherein the distal tip further includes a flange (300) with a groove (302) formed therein extending from a proximal end of the distal tip, and the distal capsule further includes a distal end (134) configured to interlock with the groove of the flange of the distal tip.

3. The delivery system of claim 2, wherein the distal end of the distal capsule further comprises an interlocking flange (134A) configured to be received by the groove of the flange of the distal tip.

4. The delivery system of claim 3, wherein the distal end of the distal capsule further includes an interlock collar (138) overlapping the distal end for securing the interlocking flange to the distal end of the distal capsule; and/or wherein the interlocking flange comprises a sloped face (300A) for sliding interaction with the flange of the distal tip, such that the flange of the distal tip is displaced from a longitudinal axis upon contacting the sloped face during the sliding interaction.

5. The delivery system of any preceding claim, wherein the removable sleeve includes a plurality of tab protrusions (202) for facilitating end user interaction; or wherein the removable sleeve includes at least one tab protrusion for facilitating end user interaction, and at least one perforation (204) disposed along a length of the removable sleeve for removal of the removable sleeve from the overlap region.

6. The delivery system of any preceding claim, wherein the distal end of the proximal capsule comprises a proximal overlap collar (130A) extending distally, and the proximal end of the distal capsule comprises a distal overlap collar (132A) extending proximally, and the proximal overlap collar is configured to overlap with the distal overlap collar to form the overlap region; and optionally wherein the proximal overlap collar comprises a first thickness, and the distal overlap collar comprises a second thickness, such that a profile of the overlap region created by the overlapping of the proximal overlap collar and the distal overlap collar is no greater than a profile of a body portion of the proximal capsule and/or a body portion of the distal capsule.

7. The delivery system of any preceding claim, wherein the outer sheath is translatable between an open configuration where the proximal capsule and the distal capsule are separated from each other, and a closed configuration where the distal end of the proximal capsule and the proximal end of the distal capsule are connected to each other forming the overlap region.

8. The delivery system of any preceding claim
wherein the distal end (134) of the distal capsule (132) is configured to interlock with the distal tip.

9. The delivery system of any preceding claim, wherein the distal tip is translatable between a loading configuration and a closed configuration, such that the distal tip is separated from the distal end of the distal capsule in the loading configuration, and the distal tip is secured to the distal end of the distal capsule in the closed configuration.

## Patentansprüche

1. Abgabesystem (100) zum Abgeben einer Vorrichtung (150) an ein Körperlumen, das Abgabesystem umfassend:
eine Innenwelle (114), die mit einer distalen Spitze (118) gekoppelt ist;
eine Außenhülle (126), die über der Innenwelle gleitend angeordnet und konfiguriert ist, um die Vorrichtung in einer radial komprimierten Konfiguration zu halten, wobei die Außenhülle eine proximale Kapsel (130) mit einem distalen Ende (131) und eine distale Kapsel (132) mit einem proximalen Ende (133) aufweist, wobei das distale Ende der proximalen Kapsel konfiguriert ist, um mit dem proximalen Ende der distalen Kapsel zu überlappen, um einen Überlappungsbereich (136) auszubilden; und
eine abnehmbare Hülse (200), die konfiguriert ist, um die proximale Kapsel in dem Überlappungsbereich mit der distalen Kapsel zu koppeln.

2. Abgabesystem nach Anspruch 1, wobei die distale Spitze ferner einen Flansch (300) mit einer darin ausgebildeten Nut (302) einschließt, die sich von einem proximalen Ende der distalen Spitze erstreckt, und die distale Kapsel ferner ein distales Ende (134) einschließt, das konfiguriert ist, um sich mit der Nut des Flansches der distalen Spitze zu verriegeln.

3. Abgabesystem nach Anspruch 2, wobei das distale Ende der distalen Kapsel ferner einen Verriegelungsflansch (134A) umfasst, der konfiguriert ist, um durch die Nut des Flansches der distalen Spitze aufgenommen zu werden.

4. Abgabesystem nach Anspruch 3, wobei das distale Ende der distalen Kapsel ferner einen Verriegelungsbund (138) einschließt, der das distale Ende überlappt, zum Sichern des Verriegelungsflansches an dem distalen Ende der distalen Kapsel; und/oder wobei der Verriegelungsflansch eine geneigte Fläche (300A) für ein gleitendes Zusammenwirken mit dem Flansch der distalen Spitze derart umfasst, dass der Flansch der distalen Spitze bei einem Kontaktieren der geneigten Fläche während des gleitenden Zusammenwirkens von einer Längsachse verlagert wird.

5. Abgabesystem nach einem der vorstehenden Ansprüche, wobei die abnehmbare Hülse eine Vielzahl von Laschenvorsprüngen (202) zum Erleichtern einer Endbenutzerinteraktion einschließt; oder wobei die abnehmbare Hülse mindestens einen Laschenvorsprung zum Erleichtern der Endbenutzerinteraktion und mindestens eine Perforation (204) einschließt, die entlang einer Länge der abnehmbaren Hülse für die Entfernung der abnehmbaren Hülse aus dem Überlappungsbereich angeordnet ist.

6. Abgabesystem nach einem der vorstehenden Ansprüche, wobei das distale Ende der proximalen Kapsel einen sich distal erstreckenden proximalen Überlappungsbund (130A) umfasst, und das proximale Ende der distalen Kapsel einen sich proximal erstreckenden distalen Überlappungsbund (132A) umfasst, und der proximale Überlappungsbund konfiguriert ist, um mit dem distalen Überlappungsbund zu überlappen, um den Überlappungsbereich auszubilden; und optional wobei der proximale Überlappungsbund eine erste Dicke umfasst und der distale Überlappungsbund eine zweite Dicke derart umfasst, dass ein Profil des Überlappungsbereichs, der durch das Überlappen des proximalen Überlappungsbunds und des distalen Überlappungsbunds erzeugt wird, nicht größer als ein Profil eines Körperabschnitts der proximalen Kapsel und/oder eines Körperabschnitts der distalen Kapsel ist.

7. Abgabesystem nach einem der vorstehenden Ansprüche, wobei die Außenhülle zwischen einer offenen Konfiguration, in der die proximale Kapsel und die distale Kapsel voneinander getrennt sind, und einer geschlossenen Konfiguration, in der das distale Ende der proximalen Kapsel und das proximale Ende der distalen Kapsel miteinander verbunden sind, wobei der Überlappungsbereich ausgebildet wird, verschiebbar ist.

8. Abgabesystem nach einem der vorstehenden Ansprüche
wobei das distale Ende (134) der distalen Kapsel (132) konfiguriert ist, um sich mit der distalen Spitze zu verriegeln.

9. Abgabesystem nach einem der vorstehenden Ansprüche, wobei die distale Spitze zwischen einer Beladungskonfiguration und einer geschlossenen Konfiguration derart verschiebbar ist, dass die distale Spitze in der Beladungskonfiguration von dem distalen Ende der distalen Kapsel getrennt ist und die distale Spitze in der geschlossenen Konfiguration an dem distalen Ende der distalen Kapsel gesichert ist.

## Revendications

1. Système d'administration (100) destiné à administrer un dispositif (150) à une lumière corporelle, le système d'administration comprenant :
une tige intérieure (114) accouplée à une pointe distale (118) ;
une gaine extérieure (126) disposée de manière coulissante sur la tige intérieure conçue pour maintenir le dispositif dans une configuration radialement comprimée, la gaine extérieure ayant une capsule proximale (130) avec une extrémité distale (131), et une capsule distale (132) avec une extrémité proximale (133), l'extrémité distale de la capsule proximale étant conçue pour chevaucher l'extrémité proximale de la capsule distale pour former une région de chevauchement (136) ; et
un manchon amovible (200) conçu pour accoupler la capsule proximale à la capsule distale au niveau de la région de chevauchement.

2. Système d'administration selon la revendication 1, dans lequel la pointe distale comporte en outre une bride (300) avec une rainure (302) formée dans celle-ci s'étendant à partir d'une extrémité proximale de la pointe distale, et la capsule distale comporte en outre une extrémité distale (134) conçue pour s'emboîter dans la rainure de la bride de la pointe distale.

3. Système d'administration selon la revendication 2, dans lequel l'extrémité distale de la capsule distale comprend en outre une bride d'emboîtement (134A) conçue pour être reçue par la rainure de la bride de la pointe distale.

4. Système d'administration selon la revendication 3, dans lequel l'extrémité distale de la capsule distale comporte en outre un collet d'emboîtement (138) chevauchant l'extrémité distale pour fixer la bride d'emboîtement à l'extrémité distale de la capsule distale ; et/ou dans lequel la bride d'emboîtement comprend une face inclinée (300A) pour une interaction coulissante avec la bride de la pointe distale, de telle sorte que la bride de la pointe distale est déplacée par rapport à un axe longitudinal lors du contact avec la face inclinée pendant l'interaction coulissante.

5. Système d'administration selon l'une quelconque revendication précédente, dans lequel le manchon amovible comporte une pluralité de saillies de languette (202) pour faciliter l'interaction de l'utilisateur final ; ou dans lequel le manchon amovible comporte au moins une saillie de languette pour faciliter l'interaction de l'utilisateur final, et au moins une perforation (204) disposée le long d'une longueur du manchon amovible pour le retrait du manchon amovible de la région de chevauchement.

6. Système d'administration selon l'une quelconque revendication précédente, dans lequel l'extrémité distale de la capsule proximale comprend un collet de chevauchement proximal (130A) s'étendant distalement, et l'extrémité proximale de la capsule distale comprend un collet de chevauchement distal (132A) s'étendant proximalement, et le collet de chevauchement proximal est conçu pour chevaucher le collet de chevauchement distal afin de former la région de chevauchement ; et éventuellement dans lequel le collet de chevauchement proximal comprend une première épaisseur, et le collet de chevauchement distal comprend une seconde épaisseur, de telle sorte qu'un profil de la région de chevauchement créée par le chevauchement du collet de chevauchement proximal et du collet de chevauchement distal n'est pas supérieur à un profil d'une partie de corps de la capsule proximale et/ou d'une partie de corps de la capsule distale.

7. Système d'administration selon l'une quelconque revendication précédente, dans lequel la gaine extérieure peut être déplacée entre une configuration ouverte où la capsule proximale et la capsule distale sont séparées l'une de l'autre, et une configuration fermée où l'extrémité distale de la capsule proximale et l'extrémité proximale de la capsule distale sont reliées l'une à l'autre, formant la région de chevauchement.

8. Système d'administration selon l'une quelconque revendication précédente
dans lequel l'extrémité distale (134) de la capsule distale (132) est conçue pour s'emboîter avec la pointe distale.

9. Système d'administration selon l'une quelconque revendication précédente, dans lequel la pointe distale peut être déplacée entre une configuration de chargement et une configuration fermée, de telle sorte que la pointe distale est séparée de l'extrémité distale de la capsule distale dans la configuration de chargement, et la pointe distale est fixée à l'extrémité distale de la capsule distale dans la configuration fermée.
